# DEMANDE DE BREVET EUROPEEN

(11) **EP 3 486 333 A1**
(43) Date de publication de la demande: **22.05.2019**
(21) Numéro de dépôt: 18207583.8
(22) Date de dépôt: 21.11.2018
(51) Int. Cl.: C12Q 1/6883

(54) **MÉTHODE DE DÉTERMINATION DE LA SUSCEPTIBILITÉ INDIVIDUELLE À DÉVELOPPER UNE MYOFASCIITE À MACROPHAGES PERSISTANTE**

(30) Priorité: 21.11.2017 FR 1761005
(71) Demandeur: Assistance Publique - Hôpitaux de Paris, 75004 Paris (FR); Association Francaise Contre Les Myopathies, 75013 Paris (FR); Université de Nice Sophia Antipolis, 06100 Nice (FR); Centre National de la Recherche Scientifique (CNRS), 75016 Paris (FR); Université Paris-Est Créteil Val de Marne, 94000 Créteil (FR); Institut National de la Santé et de la Recherche Médicale (INSERM), 75013 Paris (FR)
(72) Inventeur: GHERARDI, Romain, 75005 Paris (FR); MOGRABI, Baharia, 06300 Nice (FR); AUTHIER, François-Jérôme, 91230 Montgeron (FR)
(74) Mandataire: Regimbeau

(57) **Abrégé**

L'invention a pour objet une méthode permettant de prévoir le risque de développer une myofasciite à macrophages persistante ou l'adaptation d'un traitement d'un sujet humain susceptible de développer une myofasciite à macrophages persistante comprenant la détermination de la présence ou l'absence d'au moins un polymorphisme nucléotidique dans un échantillon biologique choisi parmi rs61746812 du gène AGT2A, rs2373927 du gène ATG9B, rs3734114 ou rs111249277 du gène ATG10, rs157397 du gène ULK2, rs4958847 du gène IGRM, ou rs6087598 du gène MAP1LC3A.

## Description

### Domaine

La présente invention se rapporte au domaine de la vaccination et plus particulièrement l'approche vaccinale utilisant des adjuvants aluminiques.

### Introduction

Les vaccins sont administrés à des sujets, soit dans le but de protéger lesdits sujets contre une maladie en stimulant son système immunitaire avant l'apparition d'une maladie (vaccins préventifs), soit pour aider le patient à lutter contre une maladie (vaccins thérapeutiques). Nous nous intéresserons plus particulièrement aux vaccins dits préventifs qui sont administrés à des sujets sains, contrairement aux médicaments conventionnels à visée curatives, et aux vaccins thérapeutiques. Les vaccins nécessitent une maîtrise accrue de leur sécurité pour assurer un minimum d'effets secondaires. Les vaccins sont généralement composés d'un ou plusieurs antigènes et d'un adjuvant, permettant de stimuler l'immunité innée et ainsi d'activer les cellules permettant d'induire la réponse immunitaire adaptative et donc stimuler la réponse immunitaire vis-à-vis de l'antigène. Parmi les adjuvants, les sels d'aluminium (e.g. l'hydroxyde d'aluminium (AlOOH), le hydroxy-phosphate d'aluminium, etc.) constituent la principale classe d'adjuvants autorisés pour les vaccins humains et animaux au niveau mondial et sont présents dans les formulations vaccinales depuis les années 1930. Cependant, au vu des manifestations cliniques associées à la vaccination chez certains individus telle que l'encéphalomyélite myalgique ou syndrome de fatigue chronique (EM/SFC), la sécurité des adjuvants aluminiques est aujourd'hui remise en question (Agmon-Levin *et al.,* 2004).

En effet, il a été démontré qu'une faible proportion de sujets vaccinés a développé une lésion histopathologique de longue durée, nommée myofasciite à macrophages persistante, qui est associée à une longue biopersistance de l'adjuvant aluminique dans les cellules immunitaires de type monocyte/macrophage (Authier *et al.,* 2003). Cette lésion biopersistante peut être détectée jusqu'à plus de 12 ans après l'immunisation, par biopsie du muscle, site d'immunisations antérieures. Ladite biopsie atteste de la persistance à long terme d'agrégats d'hydroxyde d'aluminium dérivés des vaccins dans le cytoplasme des macrophages au site d'immunisations antérieures (Gherardi *et al.,* 2001).

Chez les sujets vaccinés, la présence d'une myofasciite à macrophages persistante est souvent associée au développement d'arthromyalgies, de fatigue et de troubles cognitifs (Gherardi & Authier, 2003 ; Couette *et al.,* 2009 ; Passeri *et al.,* 2011 ; Van der Gucht *et al*., 2016). De telles manifestations peuvent être chroniques et/ou associées entre elles, donnant lieu à une affection systémique invalidante : le syndrome de fatigue chronique/encéphalo-myélite myalgique (SFC/EM). Cependant, de façon plus générale, le développement d'une myofasciite à macrophages persistante pourrait survenir après toute injection d'aluminium, telle que l'injection des adjuvants aluminiques sous-cutanée en combinaison avec des allergènes lors des manoeuvres de désensibilisation.

La toxico-cinétique de l'AlOOH, considéré comme le sel d'aluminium de référence, dépend de la taille des agrégats d'adjuvant : seuls les petits agrégats de taille bactérienne, 1-3 µm, plus facilement phagocytés, sont responsables d'une élévation de l'Al cérébral et de troubles du comportement à 6 mois chez la souris (Crépeaux *et al.,* 2016). Ces résultats soulignent le rôle critique du traitement cellulaire des particules dans l'intolérance aux adjuvants de type sel d'aluminium.

Cependant, à ce jour, aucun facteur n'a été identifié permettant de déterminer le risque d'un individu de développer une myofasciite à macrophages persistante suite à une exposition à des agents toxiques à base d'aluminium, tels que les adjuvants aluminiques.

Étant donné un extrême déséquilibre entre le nombre d'individus vaccinés et le nombre relativement faible de cas de myofasciite à macrophages persistante, l'OMS a émis l'hypothèse que la myofasciite à macrophages persistante pourrait refléter une prédisposition de certains individus à ne pas éliminer l'adjuvant de leur organisme (WHO, 1999). Ces prédispositions pourraient dépendre de caractéristiques internes (e.g. au niveau génétique) ou externes (e.g. l'exposition préalable d'un individu à des composés infectieux ou toxiques particuliers). Alors que les facteurs de risque de nature génétique ne peuvent être changés, les facteurs de risque environnementaux sont évitables. Ainsi, il est nécessaire d'identifier les individus ayant une prédisposition génétique afin de leur permettre de se prémunir contre les facteurs environnementaux qui les menacent et plus particulièrement leur éviter de développer des lésions histopathologiques de longue durée telles que la myofasciite à macrophage.

Par conséquent, il existe un besoin d'identifier de tels facteurs de risque individuels ainsi que d'établir une méthode de détermination du risque de développer une myofasciite à macrophages persistante. Comme la majorité des individus aura plusieurs vaccinations au cours de la vie, pour permettre un screening du plus grand nombre d'individus, ladite méthode doit être simple, rapide et routinière. Ladite méthode doit également être peu couteuse.

La myofasciite à macrophages persistante est aujourd'hui uniquement identifiée par une biopsie du muscle, il existe donc un besoin d'une méthode non-invasive permettant de diagnostiquer des individus atteints d'une myofasciite à macrophages persistante. Une telle méthode facilitera le diagnostic de la myofasciite à macrophages persistante. En effet, les individus ne seront pas obligés de se rendre dans un des centres spécialisés dans les biopsies musculaires qui deviennent de plus en plus rares tant en France qu'à l'étranger. Une telle méthode devra donc être plus simple et moins couteuse. Elle évitera en outre, les biopsies faussement négatives car réalisée à distance des sites exacts d'injections vaccinales antérieures, et donc la répétition de biopsies invasives.

Il existe enfin un besoin d'une méthode permettant d'adapter le soin d'un individu ayant un risque de développer une myofasciite à macrophages persistante, en particulier vis-à-vis de la vaccination ou de manoeuvres de désensibilisation par injection sous-cutanée d'allergènes combinés à des adjuvants aluminiques. Une telle méthode permettra de réduire le nombre de nouveau cas de myofasciite à macrophages persistante.

### Description

La présente invention a pour premier objet une méthode permettant de prévoir le risque de développer une myofasciite à macrophages persistante chez un sujet humain. Les inventeurs ont de façon surprenante identifié des mutations génétiques qui sont spécifiquement associées avec une myofasciite à macrophages persistante. La méthode de l'invention est avantageuse car elle est simple, basée sur un nombre réduit de variations au niveau génétique. De façon particulièrement avantageuse, ladite méthode est peu couteuse et peut être effectuée de manière routinière dans les laboratoires.

Par « *myofasciite à macrophages persistante* » on entend une lésion histopathologique inflammatoire du muscle due à une infiltration des cellules appartenant à la lignée macrophagique dans les tissus conjonctifs recouvrant l'ensemble du muscle (l'epimysium), les faisceaux de fibres musculaires (le périmysium) et/ou les fibres musculaires (l'endomysium). Ces cellules de type macrophage infiltrées contiennent des inclusions cristallines osmiophiles en microscopie électronique correspondant à l'hydroxyde d'aluminium utilisé comme adjuvant vaccinal.

Une myofasciite à macrophages est en effet induite par les sels d'aluminium utilisés dans la plupart des vaccins en tant qu'adjuvant, pour renforcer la réponse immunologique, ou suite à des manoeuvres de désensibilisation par injection sous-cutanée d'allergènes combinés à des adjuvants aluminiques. Ces lésions histopathologiques sont toujours localisées dans le site de vaccinations antérieures, habituellement le muscle deltoïde. Elles peuvent être détectées chez l'adulte comme chez l'enfant. La myofasciite à macrophages est considérée comme étant « *persistante* » lorsqu'une lésion histopathologique est présente au-delà de 18 mois après la vaccination la plus récente ou, de façon plus générale, 18 mois après l'injection d'un adjuvant aluminique.

La myofasciite à macrophages persistante est associée à diverses manifestations cliniques, comprenant des douleurs musculaires et/ou articulaires, une fatigue prolongée, et des troubles cognitifs, et à titre d'exemple des troubles de l'attention, de la mémoire et du sommeil, se rapprochant ainsi au syndrome de fatigue chronique/encéphalite myalgique.

Par « *risque de développer une myofasciite à macrophages persistante* » on entend la possibilité accrue qu'un individu développe une myofasciite à macrophages persistante après l'injection d'adjuvants aluminiques. Selon ses caractéristiques génétiques, un individu peut présenter une susceptibilité augmentée ou au contraire diminuée à développer une myofasciite à macrophages persistante.

Le risque de développer une myofasciite à macrophages persistante est déterminé par la présence d'au moins un polymorphisme nucléotidique (SNP) et augmente avec le nombre de SNPs présents. Le risque peut également être spécifiquement déterminé par la présence d'une combinaison particulière de certains SNPs. Le risque peut aussi être déterminé par une combinaison de ces deux éléments (e.g. selon le nombre et la combinaison de SNPs). Le risque peut être exprimé en pourcentage. De préférence, le risque est d'au moins 50%, 60%, 70%, 90% ou supérieur à 90%. Le risque peut être exprimé en tant qu'un rapport des cotes (aussi appelé OR pour « *odds ratio* » ; voir par exemple Garner et al., 2007 ; Jakobsdottir et al., 2009). Un rapport de cotes supérieur à 1 indique qu'il y a un risque de développer une myofasciite à macrophages persistante associé à la présence d'au moins un SNP.

Selon un mode préféré, la méthode permettant de prévoir le risque de développer une myofasciite à macrophages persistante chez un sujet comprend la détermination de la présence ou l'absence d'au moins un polymorphisme nucléotidique (SNP) dans un échantillon biologique.

Par « *sujet* », on entend au sens de la présente invention un mammifère, de préférence un humain, quel que soit son âge. Ainsi, le sujet peut être par exemple un adulte ou un enfant. Par « *adulte »,* on entend un individu dont l'âge est égal ou supérieur à 16 ans. Par « *enfant* », on entend un individu dont l'âge est inférieur à 16 ans, plus particulièrement des nourrissons à partir de la naissance jusqu'à 1 an, des enfants de 1 à 8 ans, 8 à 12 ans, et de 12 à 16 ans. Selon un mode préférentiel, le sujet est un enfant dont l'âge est compris entre la naissance et 1 mois, 2 mois, 4 mois, 6 mois, 9 mois, 1 an, 15 mois, 18 mois, 2 ans, 4 ans, 6 ans, 8 ans 10 ans, ou 12 ans d'âge. Selon un mode plus préférentiel, le sujet n'a pas encore reçu d'injection comprenant au moins un adjuvant aluminique. Selon un mode encore plus préférentiel, le sujet n'a pas encore été vacciné par un vaccin comprenant au moins un adjuvant aluminique.

Par « *échantillon biologique* » on entend un échantillon obtenu à partir d'un sujet humain comportant des acides nucléiques. Un échantillon peut comprendre des tissus et/ou des fluides biologiques. De tels échantillons peuvent être obtenu *in vitro, ex vivo* ou *in vivo.* À titre d'exemple non-limitatif, l'échantillon biologique peut être choisi parmi les tissus, les organes, les cellules, ou toute fraction isolée d'un sujet humain. L'échantillon biologique peut également être choisi parmi les fluides biologiques comprenant de façon non-limitative le sang, le plasma, la lymphe, la salive, l'urine, les selles, les larmes, la sueur, le sperme, ou le fluide cérébrospinal, synovial, pleural, péritonéal, ou péricardique, ainsi que toute fraction de ceux-ci. L'échantillon biologique peut aussi comprendre des extraits obtenus desdits fluides biologiques.

Selon un mode de réalisation préférentiel, l'échantillon biologique est un fluide biologique. De façon plus préférentielle, l'échantillon biologique est du sang, du plasma, de la lymphe ou de la salive. De façon encore plus préférentielle, l'échantillon biologique est du sang ou du plasma.

Ledit échantillon peut être obtenu par toute technique connue dans l'art antérieur. L'échantillon peut également être prétraité afin de préserver l'intégrité des acides nucléiques et/ou de les rendre plus accessible lors d'une analyse ultérieure. À titre d'exemple, l'échantillon peut être traité par des anti-nucléases. L'échantillon peut également subir des étapes de lyse (e.g. lyse chimique, mécanique, ou enzymatique), de centrifugation, de purification, etc. pour faciliter l'accès aux acides nucléiques et/ou de les concentrer.

Par « *polymorphisme nucléotidique » ou* « *SNP* » on entend une mutation ponctuelle isolée dans le génome d'un individu. Un SNP est donc une variation stable de la séquence d'ADN génomique portant sur une seule base. Un SNP selon l'invention peut avoir ou ne pas avoir d'implication fonctionnelle. Chaque SNP selon l'invention définit un locus unique. Les SNPs selon l'invention sont polymorphes : un même individu peut porter deux copies du même SNP (homozygote) ou deux SNPs différents (hétérozygote) au même locus.

De façon générale, un SNP a deux allèles différents (choisi parmi A, C, T, et G si l'acide nucléique est de l'ADN) ; il s'agit donc d'un SNP bi-allélique. Cependant, un SNP peut également avoir trois ou quatre allèles différents. De préférence, les allèles d'un SNP sont présents dans des proportions différentes dans une population donnée. Ils peuvent être en déséquilibre de liaison. Les SNPs de la présente demande sont exprimés selon le numéro de référence (rs) attribué par la base de données du NCBI (https://www.ncbi.nlm.nih.gov/snp).

Par « *allèle* » on entend une variante dans la séquence nucléotidique d'un locus. À titre d'exemple, la base nucléotidique à un locus d'un SNP bi-allélique peut être soit l'allèle C soit l'allèle A.

Un SNP, et donc l'allèle correspondant, peut être localisé à l'intérieur d'une région codante d'un gène, dans la région non-codante d'un gène, ou dans la région intergénique des gènes. Lorsqu'il est localisé à l'intérieur d'un gène, le SNP peut modifier la séquence en acides aminés de la protéine codée par ledit gène grâce à la redondance du code génétique. Un SNP modifiant la séquence d'acide aminé est appelé « *non-synonyme* ». La présence d'un tel SNP entraine un défaut qualitatif de la protéine codée par le dit gène, en impactant sa stabilité (e.g. en entrainant la dégradation), l'activité (e.g. en modifiant le site actif), la taille et/ou la conformation de la protéine « anormale » mutée, etc. Le SNP peut également influencer les interactions de ladite protéine mutée avec d'autres protéines, acides nucléiques, etc, et/ou l'expression du gène lui-même (e.g. selon le type de codon).

Lorsqu'un SNP est présent dans la région codante mais ne modifie pas l'acide aminé, il s'agit d'une variante « *synonyme* » ou silencieuse. Un tel SNP ne modifie pas la structure ni la fonction de ladite protéine. Il peut cependant conduire à des niveaux d'expression variables du gène lui-même, par exemple, en réduisant la traduction (e.g. selon la fréquence d'utilisation des ARN de transfert correspondant au codon altéré). Un SNP synonyme peut aussi moduler la stabilité (e.g. en affectant la liaison d'un microARN), et/ou la structure de l'ARN.

Enfin, un SNP présent dans une région non-codante (e.g. dans une région promotrice, dans un intron ou dans une région 3') peut influencer l'épissage d'un gène ou son niveau d'expression. Par exemple, un tel SNP peut influencer les interactions entre les régions non-codantes et les facteurs de transcription, moduler l'expression et/ou la stabilité des séquences d'ARN non-codantes, influencer la liaison des miARN, etc.

Plus particulièrement, ces SNP silencieux ou présents dans les régions non codantes peuvent être liés à des défauts quantitatifs de la protéine et sont dénommés « *locus quantitatif d'expression (eQTL)* ». Transmissibles, ils prédisposent, au-dessous ou au-dessus d'un seuil d'expression, à certaines situations pathologiques (« *trait* »).

Parmi les SNPs associés à des maladies, un grand nombre sont connus dans les gènes de l'autophagie. À titre d'exemple, des SNPs dans les gènes ATG16L1 et IRGM, impliqués dans la régulation de l'autophagie, sont associées aux maladies inflammatoires de l'intestin comme la maladie de Crohn (Brest et al., Curr Mol Med. 2010, Nat Genet 2011, Autophagy 2011). Cependant, à ce jour aucun SNP dans les gènes de l'autophagie n'est associé à la myofasciite à macrophages persistante.

De façon surprenante, les inventeurs ont identifié plusieurs SNPs dans les gènes de l'autophagie susceptibles d'être impliqués dans la biopersistance de l'adjuvant aluminique chez les patients atteints d'une myofasciite à macrophages. De façon encore plus surprenante, les inventeurs ont identifié plusieurs combinaisons de SNPs, qui sont également susceptibles d'être impliqués dans le développement d'une myofasciite à macrophages persistante.

Par « *autophagie* » on entend un processus cellulaire ubiquitaire par lequel une partie des contenus intracellulaires (e.g. des macromolécules, des organites, des pathogènes intracellulaires) est dégradée par des lysosomes. Ce processus a plusieurs fonctions. Par exemple, il préserve l'homéostasie cellulaire en modulant la biomasse intracellulaire (i.e. sa quantité) ainsi que sa qualité. Parallèlement à sa fonction catabolique, l'autophagie joue un rôle capital dans différents mécanismes liés à l'immunité. Pour faire face à l'intrusion de micro-organismes pathogènes (bactéries, virus ou parasites invasifs), la cellule est en effet capable de détourner l'autophagie de son rôle primordial « de recyclage » afin de séquestrer l'envahisseur dans une double membrane et de le dégrader. Cette élimination des pathogènes par autophagie (appelée xénophagie) constitue un des fondements de l'immunité dite « innée ». L'autophagie joue également un rôle essentiel dans l'immunité dite « acquise » ou « adaptative », c'est-à-dire une immunité spécifique permettant d'éliminer le non-soi. En effet, dans les macrophages et les cellules dendritiques, les antigènes peuvent être transférés dans des vésicules autophagiques, digérés en peptides et présentés sur des molécules du complexe majeur d'histocompatibilité (CMH) de classe II aux lymphocytes. Le mécanisme de l'autophagie repose sur la formation d'un compartiment intracellulaire bordé par une double membrane, l'autophagosome, qui, en se refermant sur lui-même va isoler des constituants du cytoplasme. Dans le processus dénommé autophagie, ce compartiment fusionne ensuite avec des lysosomes, formant l'autolysosome, ce qui permet la dégradation des contenus cytoplasmiques. L'efficacité du processus autophagique ou flux autophagique, de la séquestration à la dégradation lysosomale, dépend du niveau d'expression de chacun de ses quarante membres de sa machinerie ; comme l'attestent la perte mono-allélique du gène autophagique BECN1 dans les cancers ou les effets de dosage des variants d'ATG16L1 et d'IRGM dans La maladie inflammatoire de Crohn (Brest et al., Curr Mol Med. 2010).

Au-delà de l'autophagie qui se produit dans toutes les types cellulaires, il existe un autre processus d'autophagie non-canonique impliquant la protéine LC3 et d'autres protéines de la machinerie autophagique qui permet de dégrader des microorganismes. Ce mécanisme appelé phagocytose associée aux protéines LC3 (ou LAP pour « *LC3 associated phagocytosis* ») a été observé pour la première fois dans des cellules phagocytaires professionnelles telles que les macrophages (Sanjuan et al., 2007). La phagocytose de bactéries (*Escherichia coli* ou encore *Mycobacterium marinum*) avec des macrophages exprimant la protéine LC3-GFP induit en effet la translocation de LC3 sur les phagosomes et ce, 5 à 10 min après l'internalisation des microorganismes. Ce recrutement de LC3 semble accélérer la maturation des vésicules ainsi que la dégradation du pathogène capturé par les cellules phagocytaires.

La LAP se différencie de l'autophagie canonique puisqu'elle met en jeu un phagosome à simple membrane et non pas la formation de vésicules à doubles membranes telles que les autophagosomes (Shibutani and Yoshimori, 2014), D'un point de vue moléculaire, la LAP ne requiert pas également toutes les protéines ATG pour son fonctionnement. Par exemple, le complexe ULK1 n'est pas nécessaire alors que les protéines LC3, ATG5, ATG7 et Beclin1 sont indispensables (Martinez et al., 2011). Outre son implication dans la dégradation de pathogènes, la LAP a été aussi décrite comme étant impliquée dans l'élimination de cellules mortes (Martinez et al., 2011), la sécrétion d'IFN de type I (Henault et al., 2012) ou encore la présentation antigénique via le CMH de classe II (Romao et al., 2013).

Ainsi la cellule dispose de deux processus utilisant la machinerie autophagique pour éliminer les pathogènes (bactéries, virus, parasites) et induire une réponse immunitaire adaptative : la xénophagie et la phagocytose associée à LC3. Ces deux processus délivrent les pathogènes au lysosome pour leur dégradation et une dysrégulation de la machinerie autophagique ou de l'activité lysosomale peut nuire à la fois à l'autophagie et à la phagocytose associée à LC3.

De façon surprenante, partant de l'hypothèse que les adjuvants aluminiques se comporteraient comme des bactéries, et seraient pris en charge de la même manière dans le cadre des processus autophagique ou phagocytose associée à LC3, les inventeurs ont identifié dans 6 gènes, 7 SNPs associés à une augmentation du risque d'un individu de développer une myofasciite à macrophages persistante. De façon encore plus surprenante, les inventeurs ont identifié trois SNPs localisés dans les régions codantes, en plus de quatre SNPs de type eQTL. En effet, alors qu'il est connu que certains SNPs de type eQTL modulent fortement l'autophagie (comme ATG10 dans le cancer du poumon (Xie, 2016)) ou la phagocytose associée à LC3 (comme ATG5 dans les maladies auto-immunes telles que le lupus (Zhang, 2015)), des SNPs localisés dans les régions codantes sont très peu décrits pour avoir un tel effet. Les gènes identifiés consistent en : AGT2A, ATG9B, ATG10, ULK2, IGRM, et MAP1LC3A.

Au sens de la présente invention, par « *SNP dans un gène donné* » on entend tout SNP synonyme ou non-synonyme ainsi que tout SNP localisé dans une région intergénique ou non-codante qui est associé avec ledit gène.

Dans un mode de réalisation préféré, au moins un SNP est détecté dans au moins un des gènes choisis parmi : AGT2A, ATG9B, ATG10, ULK2, IGRM, et MAP1LC3A. Dans un mode de réalisation plus préférentiel, au moins deux SNPs sont détectés. Dans un mode de réalisation encore plus préférentiel, au moins 3, 4, 5, 6, ou 7 SNPs sont détectés. Lorsque plusieurs SNPs sont détectés, ils peuvent être présents dans le même gène ou dans des gènes différents.

Selon un autre mode de réalisation préféré, au moins un SNP est détecté parmi rs61746812 (SEQ ID NO : 2), rs2373927 (SEQ ID NO : 4), rs3734114 (SEQ ID NO : 6), rs111249277 (SEQ ID NO : 8), rs157397 (SEQ ID NO : 10), rs4958847 (SEQ ID NO : 12), et rs6087598 (SEQ ID NO : 14) (voir le Tableau 1, ci-dessous).

**Tableau 1 : SNPs associés à une augmentation du risque d'un individu de développer une myofasciite à macrophages persistante.**

| **SNP** | ***Gène*** | ***Type de SNP*** | **Changement en acide aminé** | **Réf** | **SEQ ID NO (Réf)** | **Alt** | **SEQ ID NO (Alt)** |
|---|---|---|---|---|---|---|---|
| *rs61746812* | *ATG2A* | Faux-Sens | V [Val]⁶⁶⁴ → M [Met] | ***C*** | 1 | ***T*** | 2 |
| *rs2373927* | *ATG9B* | eQTL / 3'UTR | | ***T*** | 3 | ***G*** | 4 |
| *rs3734114* | *ATG10* | Faux-Sens | S [Ser]⁶² → P [Pro] | ***T*** | 5 | ***C*** | 6 |
| *rs111249277* | *ATG10* | eQTL / Intron | | ***C*** | 7 | ***A*** | 8 |
| *rs157397* | *ULK2* | Synonyme | | ***C*** | 9 | ***T*** | 10 |
| *rs4958847* | *IRGM* | *eQTL (Promoteur)* | | ***G*** | 11 | ***A*** | 12 |
| *rs6087598* | *MAP1LC3A* | eQTL / Intron | | ***G*** | 13 | ***A*** | 14 |

| | | | | | | | |
|---|---|---|---|---|---|---|---|
| Réf : allèle de référence, non-associé à une augmentation du risque de développer une myofasciite à macrophages persistante ; Alt : allèle associé à une augmentation dudit risque | | | | | | | |

Selon un mode préférentiel, la méthode permet de prévoir le risque de développer une myofasciite à macrophages persistante chez un sujet humain comprend la détermination de la présence ou l'absence d'au moins un polymorphisme nucléotidique (SNP) dans un échantillon biologique choisi parmi rs61746812 du gène AGT2A, rs2373927 du gène ATG9B, rs3734114 ou rs111249277 du gène ATG10, rs157397 du gène ULK2, rs4958847 du gène IGRM, ou rs6087598 du gène MAP1LC3A.

Selon un mode préférentiel, la méthode permettant de prévoir le risque de développer une myofasciite à macrophages persistante chez un sujet humain comprend :
- la détermination de la présence ou l'absence d'au moins un polymorphisme nucléotidique (SNP) dans un échantillon biologique choisi parmi rs61746812 du gène AGT2A, rs2373927 du gène ATG9B, rs3734114 ou rs111249277 du gène ATG10, rs157397 du gène ULK2, rs4958847 du gène IGRM, ou rs6087598 du gène MAP1LC3A ; et
- la détermination de l'existence d'un risque lorsqu'au moins un polymorphisme nucléotidique est détecté dans l'étape précédente.

Selon un autre mode préférentiel, la méthode permettant de prévoir le risque de développer une myofasciite à macrophages persistante chez un sujet humain comprenant la détermination de la présence ou l'absence d'au moins un polymorphisme nucléotidique (SNP) chez un sujet humain, consiste à :
- déterminer la présence ou l'absence d'au moins un polymorphisme nucléotidique (SNP) dans un échantillon biologique choisi parmi rs61746812 du gène AGT2A, rs2373927 du gène ATG9B, rs3734114 ou rs111249277 du gène ATG10, rs157397 du gène ULK2, rs4958847 du gène IGRM, ou rs6087598 du gène MAP1LC3A ; et
- la détermination de l'existence d'un risque lorsqu'au moins un polymorphisme nucléotidique est détecté dans l'étape précédente.

Selon un mode préférentiel, le risque de développer une myofasciite à macrophages est déterminé selon le nombre de SNPs qui ont été identifiés.

Selon un autre mode préférentiel, la présence d'au moins un allèle choisi parmi l'allèle T de rs61746812, l'allèle G de rs2373927, l'allèle T de rs3734114, l'allèle A de rs111249277, l'allèle T de rs157397, l'allèle A de rs4958847, et l'allèle A de rs6087598 est associé à une augmentation du risque d'un individu de développer une myofasciite à macrophages. Le génotype du SNP peut être homozygote (e.g. TT pour rs61746812) ou hétérozygote (e.g. TC pour rs61746812) pour rs61746812, rs2373927, rs3734114, rs157397, et rs4958847. Le génotype du SNP est homozygote pour rs111249277 et rs6087598.

La présente invention a pour deuxième objet une méthode de diagnostic chez un sujet humain atteint d'une myofasciite à macrophages persistante comprenant :
- la détermination de la présence ou l'absence d'au moins un polymorphisme nucléotidique dans un échantillon biologique choisi parmi rs61746812 du gène AGT2A, rs2373927 du gène ATG9B, rs3734114 ou rs111249277 du gène ATG10, rs157397 du gène ULK2, rs4958847 du gène IGRM, ou rs6087598 du gène MAP1LC3A ; et
- la détermination, lorsqu'au moins un polymorphisme nucléotidique est détecté, que ledit sujet est atteint d'une myofasciite à macrophage.

De façon très avantageuse, ladite méthode permet de déterminer la présence d'une myofasciite à macrophages persistante sans intervention chirurgicale.

Selon un mode préférentiel, au moins deux SNPs sont détectés par ladite méthode de diagnostic ou par ladite méthode de détermination du risque de développer une myofasciite à macrophages persistante. De façon plus préférentielle, lorsqu'au moins deux SNPs sont détectés, au moins un de ces SNPs est localisé dans une région codante. De préférence, au moins un de ces SNPs est de type synonyme. De préférence, le SNP de type non-synonyme est localisé dans le gène ATG2. De façon encore plus préférentielle, au moins un SNP correspond à rs61746812 d'AGT2. Selon le mode de réalisation le plus préférentiel, le SNP correspond aux génotypes hétérozygote (CT) ou homozygote (TT) de rs61746812 d'AGT2.

Selon un autre mode préférentiel, les deux polymorphismes rs61746812 et rs2373927 ; ou rs61746812 et rs3734114 ; ou rs61746812 et rs111249277; ou rs61746812 et rs157397 ; ou rs61746812 et rs4958847 ; ou rs61746812 et rs6087598 ; ou rs2373927 et rs3734114 ; ou rs2373927 et rs111249277 ; ou rs2373927 et rs157397 ; ou rs2373927 et rs4958847 ; ou rs2373927 et rs6087598 ; ou rs3734114 et rs111249277 ; ou rs3734114 et rs157397 ; ou rs3734114 et rs4958847 ; ou rs3734114 et rs6087598 ; ou rs111249277 et rs157397 ; ou rs111249277 et rs4958847 ; ou rs111249277 et rs6087598 ; ou rs157397 et rs4958847 ; ou rs157397 et rs6087598 ; ou rs4958847 et rs6087598 sont détectés lors de diagnostic d'une myofasciite à macrophages persistante chez un sujet, ou lors de la détermination du risque de développer une myofasciite à macrophage persistante.

Selon un autre mode préférentiel, les trois polymorphismes rs61746812, rs2373927 et rs3734114 ; ou rs61746812, rs2373927 et rs111249277 ; ou rs61746812, rs2373927 et rs157397 ; ou rs61746812, rs2373927 et rs4958847 ; ou rs61746812, rs2373927 et rs6087598 ; ou rs61746812, rs3734114 et rs111249277 ; ou rs61746812, rs3734114 et rs157397 ; ou rs61746812, rs3734114 et rs4958847 ; ou rs61746812, rs3734114 et rs6087598 ; ou rs61746812, rs111249277 et rs157397 ; ou rs61746812, rs111249277 et rs4958847 ; ou rs61746812, rs111249277 et rs6087598 ; ou rs61746812, rs157397 et rs4958847 ; ou rs61746812, rs157397 et rs6087598 ; ou rs61746812, rs4958847 et rs6087598 ; ou rs2373927, rs3734114 et rs111249277 ; ou rs2373927, rs3734114 et rs157397 ; ou rs2373927, rs3734114 et rs4958847 ; ou rs2373927, rs3734114 et rs6087598 ; ou rs2373927, rs111249277 et rs157397 ; ou rs2373927, rs111249277 et rs4958847 ; ou rs2373927, rs111249277 et rs6087598 ; ou rs2373927, rs157397 et rs4958847 ; ou rs2373927, rs157397 et rs6087598 ; ou rs2373927, rs4958847 et rs6087598 ; ou rs3734114, rs111249277 et rs157397 ; ou rs3734114, rs111249277 et rs4958847 ; ou rs3734114, rs111249277 et rs6087598 ; ou rs3734114, rs157397 et rs4958847 ; ou rs3734114, rs157397 et rs6087598 ; ou rs3734114, rs4958847 et rs6087598 ; ou rs111249277, rs157397 et rs4958847 ; ou rs111249277, rs157397 et rs6087598 ; ou rs111249277, rs4958847 et rs6087598 ; ou rs157397, rs4958847 et rs6087598 sont détectés lors de diagnostic d'une myofasciite à macrophages persistante chez un sujet, ou lors de la détermination du risque de développer une myofasciite à macrophage persistante.

Selon un autre mode préférentiel, les quatre polymorphismes rs61746812, rs2373927, rs3734114 et rs111249277 ; ou rs61746812, rs2373927, rs3734114 et rs157397 ; ou rs61746812, rs2373927, rs3734114 et rs4958847 ; ou rs61746812, rs2373927, rs3734114 et rs6087598 ; ou rs61746812, rs2373927, rs111249277 et rs157397 ; ou rs61746812, rs2373927, rs111249277 et rs4958847 ; ou rs61746812, rs2373927, rs111249277 et rs6087598 ; ou rs61746812, rs2373927, rs157397 et rs4958847 ; ou rs61746812, rs2373927 rs157397 et rs6087598 ; ou rs61746812, rs2373927, rs4958847 et rs6087598 ; ou rs61746812, rs3734114, rs111249277 et rs157397 ; ou rs61746812, rs3734114, rs111249277 et rs4958847 ; ou rs61746812, rs3734114, rs111249277 et rs6087598 ; ou rs61746812, rs3734114, rs157397 et rs4958847 ; ou rs61746812, rs3734114, rs157397 et rs6087598 ; ou rs61746812, rs3734114, rs4958847 et rs6087598 ; ou rs61746812, rs111249277, rs157397 et rs4958847 ; ou rs61746812, rs111249277, rs157397 et rs6087598 ; ou rs61746812, rs111249277, rs4958847 et rs6087598 ; ou rs61746812, rs157397, rs4958847 et rs6087598 ; ou rs2373927, rs3734114, rs111249277 et rs157397 ; ou rs2373927, rs3734114, rs111249277 et rs4958847 ; ou rs2373927, rs3734114, rs111249277 et rs6087598 ; ou rs2373927, rs3734114, rs157397 et rs4958847 ; ou rs2373927, rs3734114, rs157397 et rs6087598 ; ou rs2373927, rs3734114, rs4958847 et rs6087598 ; ou rs2373927, rs111249277, rs157397 et rs4958847 ; ou rs2373927, rs111249277, rs157397 et rs6087598 ; ou rs2373927, rs111249277, rs4958847 et rs6087598 ; ou rs2373927, rs157397, rs4958847 et rs6087598 ; ou rs3734114, rs111249277, rs157397 et rs4958847 ; ou rs3734114, rs111249277, rs157397 et rs6087598 ; ou rs3734114, rs111249277, rs4958847 et rs6087598 ; ou rs3734114, rs157397, rs4958847 et rs6087598 ; ou rs111249277, rs157397, rs4958847 et rs6087598 sont détectés lors de diagnostic d'une myofasciite à macrophages persistante chez un sujet, ou lors de la détermination du risque de développer une myofasciite à macrophage persistante.

Selon un autre mode préférentiel, les cinq polymorphismes rs61746812, rs2373927, rs3734114, rs111249277 et rs157397 ; ou rs61746812, rs2373927, rs3734114, rs111249277 et rs4958847 ; ou rs61746812, rs2373927, rs3734114, rs111249277 et rs6087598 ; ou rs61746812, rs2373927, rs3734114, rs157397 et rs4958847 ; ou rs61746812, rs2373927, rs3734114, rs157397 et rs6087598 ; ou rs61746812, rs2373927, rs3734114, rs4958847 et rs6087598 ; ou rs61746812, rs2373927, rs111249277, rs157397 et rs4958847 ; ou rs61746812, rs2373927, rs111249277, rs157397 et rs6087598 ; ou rs61746812, rs2373927, rs111249277, rs4958847 et rs6087598 ; ou rs61746812, rs2373927, rs157397, rs4958847 et rs6087598 ; ou rs61746812, rs3734114, rs111249277, rs157397 et rs4958847 ; ou rs61746812, rs3734114, rs111249277, rs157397 et rs6087598 ; ou rs61746812, rs3734114, rs111249277, rs4958847 et rs6087598 ; ou rs61746812, rs3734114, rs157397, rs4958847 et rs6087598 ; ou rs61746812, rs111249277, rs157397, rs4958847 et rs6087598 ; ou rs2373927, rs3734114, rs111249277, rs157397 rs4958847 ; ou rs2373927, rs3734114, rs111249277, rs157397 et rs6087598 ; ou rs2373927, rs3734114, rs111249277, rs4958847 et rs6087598 ; ou rs2373927, rs3734114, rs157397, rs4958847 et rs6087598 ; ou rs2373927, rs111249277, rs157397, rs4958847 et rs6087598 ; ou rs3734114, rs111249277, rs157397, rs4958847 et rs6087598 sont détectés lors de diagnostic d'une myofasciite à macrophages persistante chez un sujet, ou lors de la détermination du risque de développer une myofasciite à macrophage persistante.

Selon un autre mode préférentiel, les six polymorphismes rs61746812, rs2373927, rs3734114, rs111249277, rs157397 et rs4958847 ; ou rs61746812, rs2373927, rs3734114, rs111249277, rs157397 et rs6087598 ; ou rs61746812, rs2373927, rs3734114, rs111249277, rs4958847 et rs6087598 ; ou rs61746812, rs2373927, rs3734114, rs157397, rs4958847 et rs6087598 ; ou rs61746812, rs2373927, rs111249277, rs157397, rs4958847 et rs6087598 ; ou rs61746812, rs3734114, rs111249277, rs157397, rs4958847 et rs6087598 ; ou rs2373927, rs3734114, rs111249277, rs157397, rs4958847 et rs6087598 sont détectés lors de diagnostic d'une myofasciite à macrophages persistante chez un sujet, ou lors de la détermination du risque de développer une myofasciite à macrophage persistante.

Selon un autre mode préférentiel, les sept polymorphismes rs61746812, rs2373927, rs3734114, rs111249277, rs157397, rs4958847 et rs6087598 sont détectés lors de diagnostic d'une myofasciite à macrophages persistante chez un sujet, ou lors de la détermination du risque de développer une myofasciite à macrophage persistante.

Selon un mode préférentiel, la méthode de détermination du risque ou de diagnostic est associée à une exposition à un composé à effet adjuvant. Selon un mode préférentiel, il s'agit d'un adjuvant aluminique.

Par « *adjuvant* » on entend un additif qui potentialise (e.g. qui améliore, prolonge, ou accélère) ou qui modifie la nature de la réponse immune humorale et/ou cellulaire en réponse à un antigène. Ledit adjuvant peut être d'origine naturelle, synthétique, ou endogène. De préférence, l'adjuvant est de type aluminique. De façon encore plus préférentielle, l'adjuvant est sélectionné parmi les sels d'aluminium oxy-hydroxyde d'aluminium ou hydroxy-phosphate d'aluminium.

Par « *adjuvant aluminique* » on entend un dérivé d'aluminium utilisé comme adjuvant, comprenant à titre non limitatif les sels d'aluminium (e.g. l'oxyde d'aluminium, l'hydroxyde d'aluminium, l'oxy-hydroxyde d'aluminium, le phosphate d'aluminium, l'alun, le sulfate d'hydroxy-phosphate d'aluminium, etc.). L'adjuvant aluminique peut être administré, par exemple, lors d'une injection, par exemple lors d'une vaccination, lors d'une désensibilisation allergique ou lors d'un traitement ou d'une chimiothérapie par des nanoparticules d'aluminium. L'adjuvant peut être administré par voie parentérale, telle que par voie intramusculaire, intradermique, intrapéritonéale, ou sous-cutanée, ou par voie topique, orale ou nasale.

Par « *vaccin* » on entend une composition comprenant au moins un immunogène qui stimule la réponse immunitaire de façon spécifique vis-à-vis dudit immunogène. Les vaccins comprennent avantageusement en outre au moins un adjuvant, notamment un adjuvant aluminique.

Par « *vaccination »* on entend l'administration d'un vaccin. La vaccination peut être prophylactique ou thérapeutique.

Par « *désensibilisation allergique* » ou « *désensibilisation* » on entend l'administration d'un allergène à des doses croissantes dans le temps. La désensibilisation peut notamment être faite par injection sous-cutanée d'une composition comprenant avantageusement en outre au moins un adjuvant, notamment un adjuvant aluminique, en plus d'au moins un allergène. La désensibilisation permet d'induire une tolérance immunitaire spécifique à un allergène. Selon un mode préférentiel, la méthode permettant de prévoir le risque de développer une myofasciite à macrophages persistante est effectuée en amont d'une exposition à un adjuvant aluminique. De façon plus préférentielle, la méthode permettant de prévoir le risque est effectuée avant toute exposition à un adjuvant aluminique. De préférence, ladite méthode est effectuée sur un échantillon biologique d'un enfant ayant reçu peu ou pas d'injections préalables contenant des adjuvants aluminiques, telles que certaines vaccinations.

Selon un mode préférentiel, la méthode de diagnostic de la présente invention est effectuée suite à une exposition à un adjuvant aluminique. Selon un autre mode préférentiel, la méthode de diagnostic est effectuée suite à une exposition à l'oxy-hydroxyde d'aluminium et/ou à l'hydroxy-phosphate d'aluminium. De façon plus préférentielle, la méthode est effectuée suite à l'administration d'un vaccin comprenant un adjuvant aluminique ou suite à l'administration d'une composition comprenant un allergène et un adjuvant aluminique dans le cadre d'une désensibilisation allergique.

La présente invention a également pour objet une méthode permettant de prévoir le risque, ou de diagnostiquer, une myofasciite à macrophages persistante chez un sujet comprenant les étapes suivantes :
a) l'isolement d'un acide nucléique dudit sujet ;
b) optionnellement, l'amplification dudit l'acide nucléique ;
c) la discrimination allélique ; et
d) la détermination de la présence ou de l'absence d'au moins un polymorphisme nucléotidique selon l'étape c).

Par « *acide nucléique* » on entend tout enchaînement linéaire de polynucléotides (e.g. d'ADN génomique, d'ADNc, d'ARN, ou d'hybride ADN-ARN), comprenant des oligonucléotides, des amorces, des sondes, des amplicons, ainsi que des fragments de type oligomère. De préférence l'acide nucléique est présent dans un échantillon biologique issu d'un sujet humain. Il peut être simple brin, double brin, ou un mélange des deux formes. L'acide nucléique peut comprendre des séquences codantes et/ou non codantes. Il peut consister en un fragment d'une molécule d'acide nucléique entière. De préférence, l'acide nucléique est de l'ADN ou de l'ADNc. De façon encore plus préférentielle, l'acide nucléique est de l'ADN génomique, ou un fragment de celui-ci.

Par « *isolement d'acide nucléique* » on entend l'obtention d'un échantillon comprenant les acides nucléiques d'un sujet humain. L'étape d'isolement peut également comprendre la *« purification* » dudit acide nucléique. Par « *purification* » on entend tout processus permettant d'augmenter la proportion de molécules d'acide nucléique vis-à-vis des autres composants d'un échantillon, ou de l'isoler des autres composants d'un échantillon. Le processus de purification peut être partiel ou total. Il peut comprendre des méthodes mécaniques, enzymatiques et/ou chimiques. À titre d'exemple, l'isolement peut comprendre une étape permettant de déstabiliser une structure cellulaire, par exemple par la lyse. L'isolement peut également comprendre une étape de dégradation d'autres composés, par exemple par la dégradation enzymatique des protéines. L'isolement peut comprendre une étape de séparation dudit acide nucléique des autres composés par centrifugation, précipitation, liaison à un support solide (e.g. à une membrane de silice, par chromatographie, à des billes magnétiques), extraction organique (e.g. par phénol-chloroforme), etc.

Par « *amplification* » on entend tout processus permettant d'augmenter la quantité d'une molécule d'acide nucléique par rapport à son niveau initial. L'amplification est dépendante de la matrice d'acides nucléiques et peut être spécifique (e.g. utilisant des amorces spécifiques correspondant à des séquences exactes, par réaction en chaîne par polymérase (PCR)) ou non-spécifique (e.g. par amplification par déplacement multiple utilisant des hexamères). Les méthodes pour réaliser une telle amplification sont bien connues de l'homme de métier.

À titre d'exemple non-limitatif, la molécule d'acides nucléiques peut être insérée dans un vecteur d'expression et amplifiée dans une cellule hôte appropriée ou par transcription *in vitro.* Un fragment d'ADN génomique peut également être obtenu par digestion enzymatique, utilisant des enzymes de restriction. De façon préférentielle, l'amplification de l'acide nucléique est faite par PCR. De façon plus préférentielle, l'amplification est faite par PCR avec des amorces permettant l'amplification d'un fragment comprenant un SNP parmi rs61746812, rs2373927, rs3734114, rs111249277, rs157397, rs4958847, ou rs6087598. De préférence, les amorces consistent en 12 à 30 nucléotides contigus.

Selon un autre mode préférentiel, l'amplification de l'acide nucléique est faite par amplification isotherme dudit SNP. De façon préférentielle, l'amplification isotherme consiste en l'amplification par déplacement multiple (SDA), l'amplification dépendante d'hélicase (HDA), l'amplification isotherme facilitée par boucle (LAMP), l'amplification basé sur la séquence d'acide nucléique (NASBA), l'amplification circulaire (RCA), la réaction d'amplification exponentielle (EXPAR), l'amplification des acides nucléiques par amorce chimérique et isotherme (ICAN), la technologie d'amplification d'ARN médiée par signale (SMART), la réaction avec amplification enzyme de coupure de brin (NEAR)) et d'autres (voir, par exemple, Asiello and Baeumner, 2011).

Par « *discrimination allélique* » on entend de façon non-limitative une méthode d'hybridation, d'incorporation nucléotidique, de ligation d'oligonucléotide, de clivage invasif, de digestion enzymatique, ou de séquençage, permettant de déterminer l'allèle d'un SNP à un locus.

Par « *hybridation* », on entend la formation d'un complexe spécifique entre deux séquences polynucléotidiques simple brin due à un appariement des bases complémentaires. Par « *formation d'un complexe spécifique* » on entend la formation d'un complexe qui est dépendant de la séquence précise au locus SNP. Lors de l'hybridation, la présence d'une erreur d'appariement au niveau de la base d'intérêt déstabilise l'interaction entre la séquence d'intérêt et la séquence complémentaire. Cette déstabilisation peut être détectée. De façon préférentielle, la déstabilisation de l'interaction empêche l'hybridation. A titre d'exemple non-limitatif, l'hybridation peut être effectuée pendant un PCR simple ou multiplex. L'hybridation peut également être effectuée à la suite d'une étape d'amplification de l'acide nucléique.

De préférence, l'hybridation a lieu entre une séquence d'intérêt, comprenant un SNP, et un oligonucléotide (e.g. une sonde ou une amorce).

Par « *sonde* » on entend un oligonucléotide marqué qui s'hybride avec une molécule d'acide nucléique ayant un allèle particulier au locus SNP. L'interaction de la sonde avec un allèle particulier au locus SNP peut ensuite être détectée. À titre d'exemple non-limitatif, une sonde peut être couplée à un marqueur fluorescent, luminescent, radioactif, chimique, enzymatique, ou électrique. La longueur d'une sonde peut être entre 8 et 50 nucléotides. De préférence, la longueur d'une sonde est entre 9 et 40 nucléotides.

Par « *amorce* » on entend un oligonucléotide qui s'hybride en amont et/ou en aval d'un locus SNP, qui permet ensuite l'amplification et/ou l'identification du locus SNP.

De préférence, la sonde ou l'amorce correspond à un allèle du SNP. Selon un mode préférentiel, l'hybridation par amorce comprend la méthode diagnostic multiplex spécifique à l'allèle (MASDA) ou une puce à ADN. Selon un mode encore plus préférentiel, l'hybridation d'une sonde comprend l'hybridation par phare moléculaire, ou sonde d'hydrolyse (e.g. Taqman), ou l'hybridation dynamique spécifique de l'allèle (DASH). De préférence, la sonde est une sonde marquée de 8 à 50 nucléotides contigus s'hybridant avec au moins un SNP choisi parmi rs61746812, rs2373927, rs3734114, rs111249277, rs157397, rs4958847, et rs6087598. De préférence, l'hybridation est faite sur un support solide. De façon encore plus préférentiel, l'hybridation est faite sur puce à ADN, plus particulièrement à base d'oligonucléotides, d'ADN, ou d'ADNc, ou par puce SNP.

Par « *incorporation nucléotidique* » on entend l'incorporation d'un nucléotide complémentaire au locus SNP. Le nucléotide peut être modifié ou marquée afin de faciliter sa détection. Un nucléotide peut être incorporé pendant le séquençage dudit locus, pendant une réaction d'amplification, par exemple de type PCR, ou lors d'une extension d'amorce. A titre d'exemple non-limitatif, un nucléotide peut être marqué par une molécule fluorescente, chimique, magnétique, radioactif. À titre d'exemple non-limitatif, un nucléotide peut être identifié par mesure de sa masse particulière. De préférence, l'incorporation nucléotidique est faite par extension d'amorce.

Par « *ligature d'oligonucléotide* » on entend la ligature entre deux oligonucléotides, l'un adjacent à l'autre, lorsque leur appariement aux bases complémentaires est parfait au site de ligature. Selon un mode préférentiel, un allèle SNP est détecté par ligature d'oligonucléotide.

Par « *clivage invasive* » on entend la formation d'une structure sensible au clivage lorsque des sondes chevauchantes s'hybrident.

Les méthodes de ligature d'oligonucléotide et de clivage invasive ne nécessitent pas d'étape d'amplification préalable obligatoire.

Par « *digestion enzymatique* » on entend la digestion d'une séquence par des enzymes de restriction dépendant de l'allèle du SNP. Les fragments de restriction peuvent ensuite être analysés sur gel, par exemple par polymorphisme de longueur des fragments de restriction. Par « *fragments de restriction* » on entend tout fragment issu d'une digestion enzymatique dans laquelle l'enzyme coupe l'ADN double brin à une séquence précise.

Selon un mode préférentiel, la discrimination allélique est faite par polymorphisme de longueur des fragments de restriction, par extension d'amorce, ou par digestion par des nucléases.

Selon un autre mode préférentiel, la discrimination allélique est faite par hybridation d'une sonde, par amplification, par séquençage, par spectrométrie de masse, par transfert sur membrane par la technique de Southern, ou par une combinaison de ces techniques.

Selon un mode préférentiel, la discrimination allélique est faite par séquençage. Par « *séquençage* » on entend une méthode permettant de déterminer la séquence d'un acide nucléique. Un grand nombre de méthodes de séquençage sont connues dans l'art antérieur. A titre d'exemple, les méthodes de séquençage comprennent le séquençage par didésoxy de Sanger ou par terminaison de chaîne, le séquençage du génome entier, le séquençage par hybridation, le pyroséquençage, l'électrophorèse capillaire, le séquençage par cycle, le séquençage par l'extension d'une seule base, le séquençage sur phase solide, le séquençage à haut débit, le séquençage massivement parallèle de signature, le séquençage par nanopore, le séquençage par microscopie électronique à transmission, le séquençage optique, la spectrométrie de masse, le séquençage 454, le séquençage par terminateur réversible marqué, le séquençage par « *paired end* » ou « *mate pair* », le séquençage par exonucléase, le séquençage par ligature (e.g. selon la technologie SOLiD), le séquençage par lecture court, le séquençage d'une seule molécule, le séquençage par dégradation chimique, le séquençage par synthèse, le séquençage parallèle en masse, le séquençage en temps réel, le séquençage par ion semi-conducteur (e.g. Ion Torrent), le séquençage multiplex de paires de gamme dimarqueurs (MS-PET), le microfluidique, ainsi que des combinaisons de ces méthodes.

Le séquençage peut être effectué sur l'ARN quand ledit SNP est localisé dans la région codante. Sinon, il est effectué sur l'ADN. De façon optionnelle, l'ADN peut être fragmenté de façon aléatoire avant le séquençage. Le séquençage des SNPs peut être réalisé par n'importe quelle technique connue dans l'art antérieur.

Selon un mode préférentiel, la discrimination allélique est faite par séquençage, plus particulièrement par séquençage direct, par ligature, par synthèse, par terminaison de chaîne, par une seule molécule en temps réel, par ion semi-conducteur, par microfluidique, par séquençage parallèle en masse, ou par pyroséquençage.

Une approche est d'utiliser une méthode permettant le génotypage quantitatif d'acides nucléiques obtenu de l'échantillon biologique avec un niveau de précision élevé. Selon un mode particulier, cette précision est obtenue par l'analyse d'un grand nombre de molécules d'acide nucléique (e.g. des millions ou des milliards) sans étape d'amplification préalable, utilisant des protocoles qui s'appuient sur les connaissances antérieures des séquences cibles (dans le cas présente, des SNPs). Selon un mode préférentiel, la méthode de séquençage parallèle en masse est utilisée. À titre d'exemple non-limitatif, ladite méthode peut être effectuée par la plateforme « *Illumina Genome Analyzer* » (Bentley et al., 2008), la plateforme Roche 454 (Margulies et al., 2005), la plateforme ABI SOLiD (McKernan et al., 2009), la plateforme Helicos de séquençage d'une molécule unique (Harris et al., 2008), le séquençage de molécule unique en temps réel (Eid et al., 2009), le séquençage Ion Torrent (WO 2010/008480; Rothberg et al., 2011), ou le séquençage par nanopore (Clarke et al., 2009).

De préférence, le séquençage parallèle en masse est fait sur un sous-ensemble aléatoire de molécules d'acide nucléique dans l'échantillon biologique.

De façon encore plus préférentielle, la méthode et le kit de la présente invention sont adaptés pour fonctionner sur un séquenceur d'ADN de type ABI PRISM(R) 377, un analyseur génétique de type ABI PRISM(R) 310, 3100, 3100-Avant, 3730, ou 3730x1, un système de type Applied Biosystems SOLiD(TM) (tout d'Applied Biosystems), un système de type Genome Sequencer 20 (de Roche Applied Science), un HiSeq 2500, un HiSeq 2000, un analyseur génomique de type IIx, un séquenceur personnel MiSeq, un HiScanSQ (tout d'Illunima), le système d'analyse génétique comprenant le « *Single Molecule Sequencer* », l' « *Analysis Engine* » et le « *Sample Loader* » (tout de HeliScope), le séquenceur de type Ion Proton™ ou Ion PGM™ (les deux d'Ion Torrent).

Le séquençage comprend également des méthodes basées sur la réaction en chaîne par polymérase (PCR), comprenant à titre d'exemple non-limitatif, la PCR quantitative ou la PCR en émulsion.

Selon un autre mode préférentiel, la discrimination allélique est faite par amplification. L'amplification comprend plus particulièrement des méthodes isothermes (e.g. telles que détailles ci-dessus) ainsi que des méthodes de type PCR.

De préférence, la discrimination allélique est faite par PCR.

La présente invention a également pour objet une méthode permettant d'identifier chaque allèle d'au moins un SNP choisi parmi rs61746812, rs2373927, rs3734114, rs111249277, rs157397, rs4958847, et rs6087598 chez un sujet comprenant les étapes suivantes :
a) l'obtention d'acide nucléique dudit sujet ; et
b) la détection de chaque allèle d'au moins un SNP choisi parmi rs61746812, rs2373927, rs3734114, rs111249277, rs157397, rs4958847, et rs6087598 selon une quelconque des méthodes décrites ci-dessus.

La présente invention a comme autre objet un kit permettant de diagnostiquer une myofasciite à macrophages persistante. Le kit permet de détecter la présence ou l'absence d'au moins un allèle d'au moins un SNP choisi parmi les SNPs du Tableau 3, la présence d'au moins un allèle d'au moins un SNP indiquant une augmentation du risque de développer un myofasciite à macrophages persistante. Dans un mode préféré, le kit comprend un réactif permettant de détecter la présence ou absence d'au moins le génotype TT ou CT de rs61746812.

Le kit comprend les réactifs nécessaires pour réaliser au moins une des méthodes précitées. Il peut comprendre, par exemple, des amorces, des sondes, des enzymes de restriction, des nucléases, des oligonucléotides reconnaissant une séquence allélique spécifique. Selon un mode préféré, le kit comprend au moins un couple d'amorces permettant l'amplification d'au moins un fragment du génome comprenant un SNP. De préférence le fragment amplifié est compris entre 50 et 500 paires de bases (pb). De façon encore plus préférentielle, le fragment amplifié est compris entre 50 et 150 paires de bases. Selon un autre mode de réalisation particulier, le kit comprend au moins une sonde complémentaire à un allèle du SNP. De préférence, la longueur de la sonde est comprise entre 8 et 50 pb. De façon plus préférentielle, la sonde est entre 9 et 40 pb en longueur. De façon encore plus préférentielle la sonde comprend entre 8 et 50 nucléotides contigus s'hybridant avec au moins un allèle d'un SNP choisi parmi rs61746812, rs2373927, rs3734114, rs111249277, rs157397, rs4958847, et rs6087598.

La présente invention a également pour objet une méthode pour l'adaptation d'un traitement d'un sujet humain susceptible de développer une myofasciite à macrophages persistante, comprenant :
- la détermination d'un risque de développer la myofasciite à macrophages ; et
- l'adaptation du traitement dudit sujet.

Selon un mode de réalisation préféré, l'adaptation du traitement comprend la suppression ou la diminution de l'exposition du sujet aux adjuvants aluminiques. Selon un mode de réalisation plus préféré, l'adaptation dudit traitement comprend la suppression ou la diminution de l'exposition du sujet aux sels d'aluminiums injectés. Selon un mode de réalisation plus préféré, l'adaptation dudit traitement consiste en la suppression de l'exposition du sujet aux sels d'aluminiums injectés, de préférence la suppression de l'exposition du sujet aux sels d'aluminiums injectés par voie intramusculaire ou par voie sous-cutanée. Selon un mode de réalisation préféré, l'adaptation du traitement comprend l'administration d'un médicament permettant de moduler le flux autophagique ou de la phagocytose associée à LC3. De façon encore plus préférée, le médicament permettant de stimuler l'autophagie ou la phagocytose associée à LC3 est choisi parmi : des modulateurs du flux autophagique : N-esther méthylique d'arginine (l-arginine methyl ester; L-NAME), des inhibiteurs de mTOR (rapamycine, everolimus, temsirolimus), un agoniste du récepteur imidazoline (e.g. le clonidine ou le rilmenidine), vitamine D, tamoxifen, metformin, PP242, des mimétiques de type BH3 (ABT-737), Resveratrol ; des activateurs de la phagocytose (des activateurs du Récepteur de l'EGF), des modulateurs de la transcription des genes autophagique (des inhibiteurs des histones déacetylases tels que SAHA, Trichostatin A ou des agonistes de PPAR comme les acides gras oméga 3).

Le sujet peut être exposé aux sels d'aluminiums injectés, à titre d'exemple non-limitatif, dans le cadre de la vaccination ou la désensibilisation allergique. Selon un mode de réalisation préféré, l'exposition aux sels d'aluminiums injectés se fait par vaccination ou par désensibilisation allergique. Selon un mode de réalisation plus préféré, l'adaptation dudit traitement comprend la suppression de la vaccination par injection lorsque le vaccin comprend au moins un sel d'aluminium. Selon un mode de réalisation plus préféré, l'adaptation dudit traitement comprend la suppression de la désensibilisation allergique lorsque la composition comprenant l'allergène comprend au moins un sel d'aluminium. Selon un mode préférentiel, l'adaptation dudit traitement comprend le remplacement d'au moins un vaccin comprenant au moins un sel d'aluminium par un vaccin équivalent qui ne comprend pas de sel d'aluminium. Selon un mode de réalisation plus préféré, l'adaptation dudit traitement comprend le remplacement d'au moins composition utilisée dans la désensibilisation allergique comprenant l'allergène et au moins un sel d'aluminium par une composition qui ne comprend pas de sel d'aluminium. Selon un mode préférentiel, l'adaptation dudit traitement consiste dans le remplacement d'au moins un vaccin comprenant au moins un sel d'aluminium par un vaccin équivalent qui ne comprend pas de sel d'aluminium. Selon un mode préférentiel, l'adaptation dudit traitement consiste dans le remplacement d'au moins une composition utilisée dans la désensibilisation allergique comprenant l'allergène et au moins un sel d'aluminium par une composition qui ne comprend pas de sel d'aluminium.

La présente invention a également pour objet une méthode de diagnostic et de traitement d'un sujet humain susceptible de développer une myofasciite à macrophages persistante, comprenant :
a) l'obtention d'acide nucléique dudit sujet ; et
b) la détection d'au moins allèle choisi parmi l'allèle T de rs61746812, l'allèle G de rs2373927, l'allèle T de rs3734114, l'allèle A de rs111249277, l'allèle T de rs157397, l'allèle A de rs4958847, et l'allèle A de rs6087598 et d'au moins un SNP choisi parmi rs61746812, rs2373927, rs3734114, rs111249277, rs157397, rs4958847, et rs6087598 selon une quelconque des méthodes décrites ci-dessus ;
c) la détermination du risque dudit sujet de développer une myofasciite à macrophages persistante ;
d) le traitement dudit sujet par la diminution de l'exposition du sujet aux adjuvants aluminiques, de préférence dans le cadre de la vaccination ou la désensibilisation allergique, telle que détaillée ci-dessus et/ou par l'administration d'un médicament permettant de moduler le flux autophagique ou la phagocytose associée à LC3, de préférence telle que détaillée ci-dessus.

Selon un mode préférentiel, le traitement à l'étape d) comprend le remplacement de l'administration d'au moins un vaccin comprenant au moins un sel d'aluminium par un vaccin équivalent qui ne comprend pas de sel d'aluminium. De façon alternative ou complémentaire, le traitement à l'étape d) comprend le remplacement du sel d'aluminium par un autre composé lors de la désensibilisation allergique.

Un autre objet de la présente invention comprend une méthode pour l'identification d'au moins un SNP permettant de pouvoir déterminer le risque d'un sujet de développer une myofasciite à macrophages comprenant :
a) l'identification d'au moins un SNP présent dans l'un au moins des 34 gènes de la machinerie autophagique listés dans le Tableau 2 ;
b) la détermination de la présence ou absence dudit SNP dans un groupe d'individus ayant un risque de développer une myofasciite à macrophages persistante ou déjà diagnostiqué avec une myofasciite à macrophages persistante ;
c) la détermination de la présence ou absence dudit SNP dans un groupe d'individus témoin ; et
d) la détermination d'une différence significative dans la fréquence de la présence d'au moins un SNP entre les individus ayant un risque de développer une myofasciite à macrophages persistante ou déjà diagnostiqué avec une myofasciite à macrophages persistante, et la fréquence dans les individus témoin.

Par « *individu témoin* », on entend tout individu n'ayant pas de susceptibilité particulière à développer un myofasciite à macrophages ainsi que tout individu qui pourrait être sélectionné aléatoirement dans la population sans antécédent particulier.

Par « *différence significative* » on entend tout différence entre la fréquence d'au moins allèle dans le groupe susceptibilité à, ou diagnostiquer avec une myofasciite à macrophages comparé à sa fréquence dans la groupe témoin ayant une valeur p inférieure à 0,05 lorsqu'une analyse statistique est effectuée. De préférence, la valeur p est inférieure à 0,01, inférieure à 0,001, ou inférieure à 0,0001.

Selon un mode préféré, un SNP ayant une valeur p équivalente ou inférieure à 0,05 peut être utilisé pour prévoir le risque de développer une myofasciite à macrophages persistante chez un sujet humain. De préférence, au moins deux SNPs sont présents.

### LEGENDES DES FIGURES

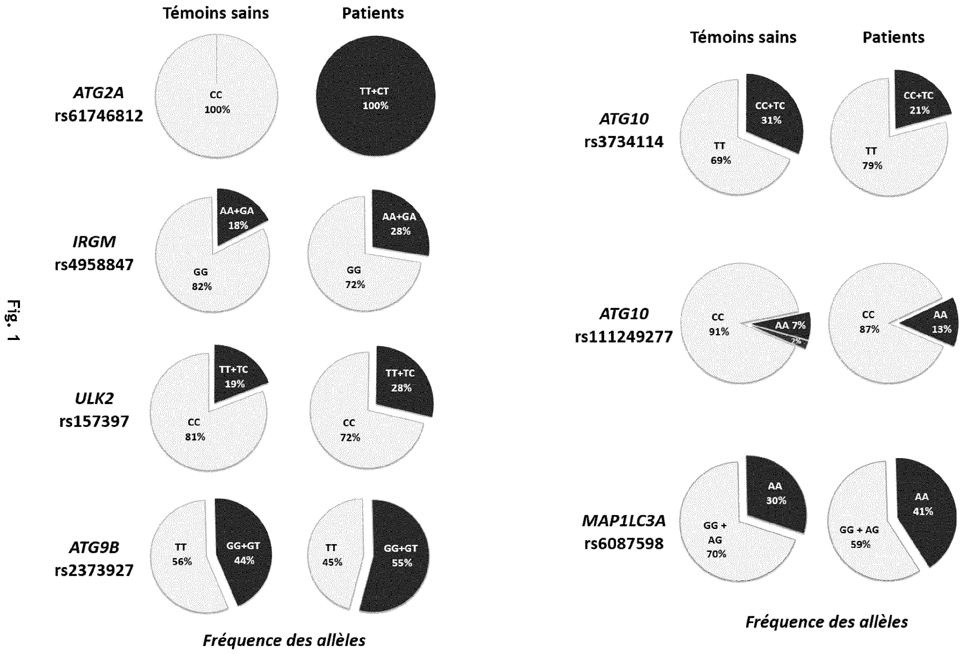

**Figure 1** **: Détermination de la fréquence allélique de 6 SNPs dans les gènes de l'autophagie.**
   La fréquence des allèles des SNPs (« *fréquence des allèles* ») dans un groupe « *sain* », ne présentant pas de signes cliniques habituellement associé à une myofasciite à macrophages persistante (« *témoins* sains »), a été comparée à leur fréquence dans un groupe de *« patients* » symptomatiques atteints d'une myofasciite à macrophages persistante. La fréquence de chaque allèle est exprimée en pourcentage ; l'allèle associé à une augmentation du risque d'un individu à développer une myofasciite à macrophages persistante est indiqué en noir. Les allèles associés à une augmentation du risque sont plus fréquents chez les patients.

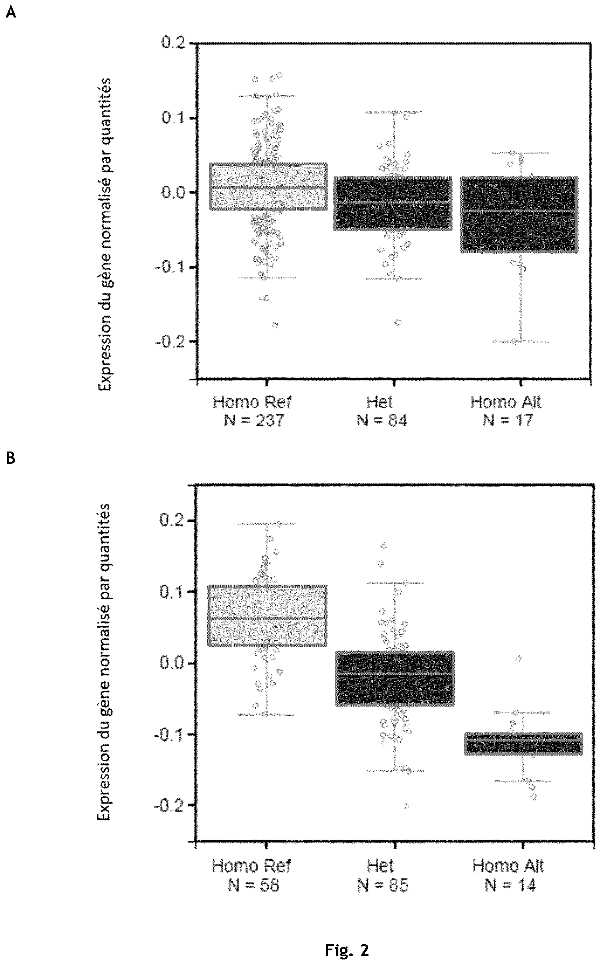

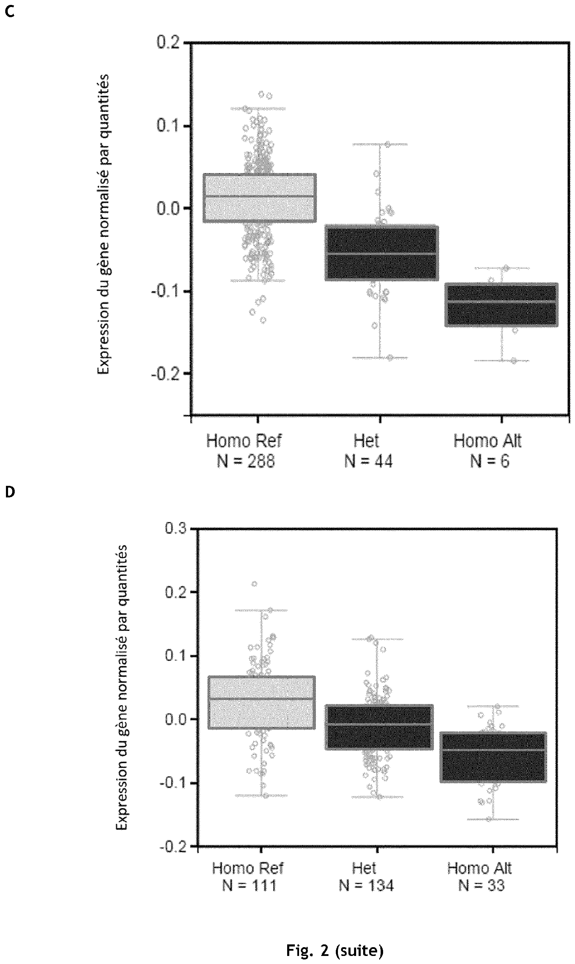

**Figure 2** **: Box-plots des SNPs de type eQTL.**
   Le graphique box plot présente les niveaux d'expression des gènes autophagiques stratifié par génotype de chaque SNP de type eQTL dans le sang ou divers tissus de 100 à 500 donneurs, sur la base de l'analyse GTEx version 6 (http://gtexportal.org/).
   (A) rs4958847 d'IGRM. L'allèle alterné est A et a été associé à un risque augmenté de MFM (P = 0.03) et d'expression d'IRGM réduite (P = 0.0000066). "Homo Ref" se réfère au génotype d'allèle de référence homozygote, GG ; "Het" se réfère au génotype hétérozygote, GA ; et "Homo Alt" se réfère au génotype allèle alternatif homozygote, AA.
   (B) rs2373927 de ATG9B. L'allèle alterné est G et a été associé à un risque augmenté de MFM (P =0.03) et d'expression d'ATG9B réduite (P = 1.2 e-20). "Homo Ref" se réfère au génotype d'allèle de référence homozygote, TT ; "Het" se réfère au génotype hétérozygote, GT ; et "Homo Alt" se réfère au génotype allèle alternatif homozygote, GG.
   (C) rs111249277 de ATG10. L'allèle alterné est A et a été associé à un risque augmenté de MFM (P =0.01) et d'expression d'ATG10 réduite (P = 2.2 e-23). "Homo Ref" se réfère au génotype d'allèle de référence homozygote, CC ; "Het" se réfère au génotype hétérozygote, CA ; et "Homo Alt" se réfère au génotype allèle alternatif homozygote, AA.
   (D) rs6087598 de MAP1LC3A. L'allèle alterné est A et a été associé à un risque augmenté de MFM (P = 0.01) et d'expression de MAP1LC3A réduite (P = 2.8e-16). "Homo Ref" se réfère au génotype d'allèle de référence homozygote, GG ; "Het" se réfère au génotype hétérozygote, AG ; et "Homo Alt" se réfère au génotype allèle alternatif homozygote, AA.
   Le niveau d'expression est normalisé par quantile sur la distribution moyenne empirique observée sur les échantillons. Pour chaque gène, le niveau d'expression a été normalisé par quantile inversé sur la distribution standard normale observée sur les échantillons (voir aussi https://www.gtexportal.org/home/documentationPage, version V6p). Le niveau d'expression est déterminé pour chaque combinaison allélique (e.g. homozygote de l'allèle le plus fréquent, hétérozygote des deux allèles, et homozygote pour l'allèle le moins fréquent).

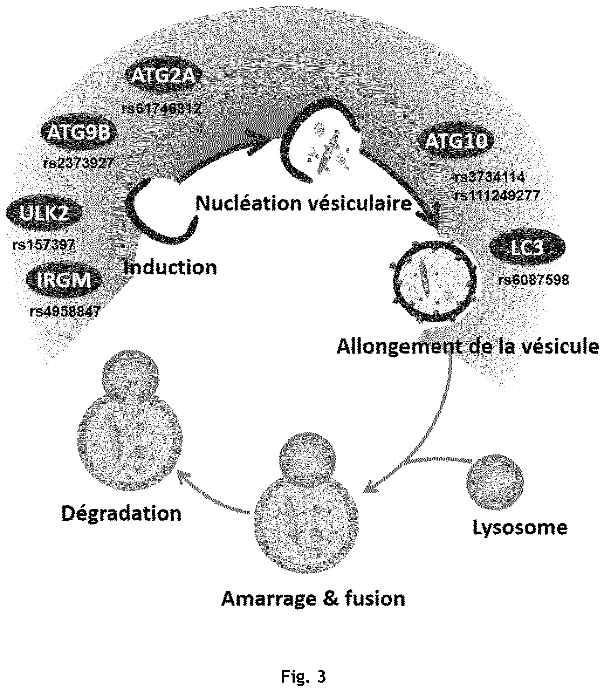

**Figure 3** **: Représentation graphique des 6 gènes impliqués dans l'autophagie.**

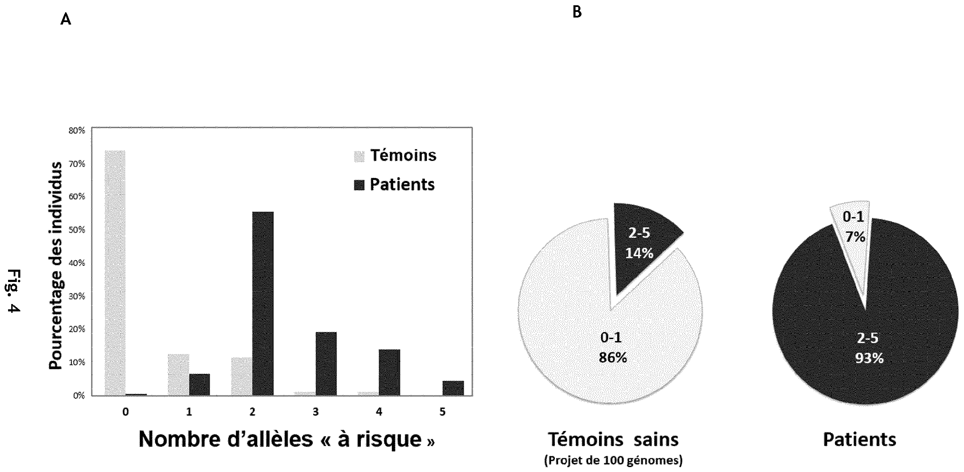

**Figure 4** **: Comparaison du nombre de SNPs présent dans une population témoin par rapport à une population de patients.** (A) Pourcentage d'individus « sains » (groupe « *témoin* sains ») ayant de 0 à 5 allèles associés à la myofasciite à macrophages persistante, comparé au pourcentage d'individus issu du groupe « *patients* » symptomatiques atteints d'une myofasciite à macrophages persistante. (B) Comparaison du pourcentage d'individus dans la population témoin (« *control* ») ayant de 0 à 1 allèle associé à la myofasciite à macrophages persistante ou de 2 à 5 allèles par rapport à une population de « *patients ».*

### EXEMPLES

L'invention est illustrée par les exemples non-limitatifs suivants. Ces enseignements comprennent des alternatives, des modifications et des équivalents, tels que pourront être apprécié par un homme de métier.

### Exemple 1 : Sélection des Patients

### Matériels et Méthodes :

365 patients ont été sélectionnés sur 5 critères d'inclusion :
(i) début des manifestations cliniques postérieur à l'administration de vaccins aluminiques ;
(ii) arthromyalgies diffuses durant plus de 6 mois et/ou fatigue inhabituelle durant plus de 6 mois et/ou plaintes cognitives invalidantes affectant l'attention et la mémoire ;
(iii) lésion de myofasciite à macrophages caractéristique à la biopsie du muscle deltoïde validée par un des centres ayant décrit l'entité (Créteil, Paris, Marseille et Bordeaux, France) ;
(iv) temps écoulé entre la dernière vaccination et la biopsie musculaire montrant la lésion de myofasciite à macrophages supérieure à 18 mois ; et
(v) absence d'autres affections pouvant expliquer les manifestations cliniques.

### Résultats :

La plupart des patients étaient de sexe féminin (70%) et d'âge moyen de 47 ans au moment de la biopsie (médiane 45 ans, extrêmes 17-74). Ils avaient reçu en moyenne 5,2 vaccins aluminiques au cours des 10 ans précédent la détection de la myofasciite à macrophages. Ils souffraient d'arthro-myalgies (86%), de fatigue chronique (78%), et d'altérations cognitives patentes (50%). Le délai moyen écoulé entre la dernière vaccination et la biopsie était de 66 mois (extrêmes 18-219).

Tous les patients ont donné leur consentement éclairé à l'étude. L'ADN génomique a été extrait de la salive par l'utilisation d'un kit de prélèvement d'ADN non invasif (technologie Oragene™) ou de fragments de muscle congelé au moment de la biopsie.

### Exemple 2 : Sélection des SNPs

103 SNPs sur 34 gènes autophagiques ont été choisis à l'aide de bases de données internationales (http://www.hapmap.org/; www.gtexportal.org/). Ils incluaient tous les SNPs précédemment cités dans PUBMED et associés à des pathologies comme les maladies inflammatoires chroniques intestinales (MICI), tous les SNPs perte-de-fonction (i.e., faux-sens, frame-shift, et variantes d'épissage), tous les SNPs non-synonymes changeant la nature de l'acide aminé, affectant probablement la fonction de la protéine, et tous les SNPs eQTL localisés dans les régions non codantes 5' promotrices, introniques et 3'.
Seuls les SNPs avec une fréquence de l'allèle mineur (MAF) comprise entre 5% et 40% dans la population européenne ont été inclus pour des raisons statistiques. Les 34 gènes et 103 SNPs analysés dans la présente étude sont détaillés dans le Tableau 2, ci-dessous.

**Tableau 2 : SNPs évalués (les 103)**

| **Nom du gène** | **Numéro d'accession du gène** | **Numéro d'accession du SNP (rs)** | **Type de variant** | **Réf** | **Alt** |
|---|---|---|---|---|---|
| AMBRA1 | NM_017749 | rs72910100 | Faux-sens | C | A |
| ATG2A | NM_015104 | rs12293826 | Faux-sens | C | T |
| | | rs148192310 | Faux-sens | C | T |
| | | rs2285347 | Faux-sens | C | T |
| | | rs35115827 | Faux-sens | G | A |
| | | rs60711419 | Faux-sens | G | A |
| | | rs61741398 | Faux-sens | G | A |
| | | rs61746812 | Faux-sens | C | T |
| | | rs77833427 | Faux-sens | C | T |
| ATG2B | NM_018036 | rs142484701 | Faux-sens | G | A |
| | | rs143695335 | Faux-sens | A | G |
| | | rs146022217 | Faux-sens | G | C |
| | | rs74719094 | Faux-sens | T | G |
| | | rs77998773 | Faux-sens | T | C |
| | | rs9323945 | Faux-sens | T | C |
| ATG3 | NM_022488 | rs143658116 | Faux-sens | T | G |
| ATG4A | NM_178270 | rs5973822 | 3'-UTR | A | G |
| | | rs79788197 | Faux-sens | A | C |
| ATG4B | NM_178326 | rs138274580 | Faux-sens | A | G |
| | | rs62190299 | eQTL ; intron | T | C |
| | | rs7601000 | Faux-sens_eQTL | T | A |
| | | rs79942484 | eQTL ; 3'-UTR | AT | DEL |
| ATG4D | NM_032885 | rs2304165 | eQTL ; synonyme | G | A |
| ATG5 | NM_004849 | rs3827644 | Intron | G | C |
| ATG7 | NM_001144912 | rs36117895 | Faux-sens | T | C |
| ATG9A | NM_001077198 | rs2276635 | Faux-sens | T | C |
| ATG9B | NM_173681 | rs2373927 | eQTL ; 5' promoteur | T | G |
| | | rs6944859 | 5' promoteur | G | T |
| | | rs111549985 | eQTL ; 5'-promoteur | C | G |
| | | rs11741569 | eQTL ; Intron | C | G |
| | | rs111249277 | eQTL ; Intron | C | A |
| | | rs1864182 | Faux-sens | G | T |
| | | rs28750741 | eQTL ; Intron | G | T |
| | | rs3734114 | Faux-sens | T | C |
| | | rs41271129 | eQTL ; Intron | A | C |
| ATG10 | NM_001131028 | rs6880209 | eQTL ; 3'-UTR | C | T |
| | | rs2407156 | 3' (ATP6AP1 L) | A | G |
| | | rs6882565 | eQTL ; Intron | G | A |
| | | rs73134722 | eQTL ; Intron | G | A |
| | | rs77182875 | eQTL ; Intron | T | A |
| | | rs10514231 | eQTL ; Intron | C | T |
| | | rs150934 | eQTL | C | T |
| | | rs186260789 | eQTL ; 5' promoteur | T | A |
| ATG12 | NR_033362 | rs74844425 | Faux-sens | T | C |
| ATG14 | NM_014924 | rs57295720 | Faux-sens | C | T |
| ATG16L1 | NM_030803 | rs2241880 | Faux-sens | T | C |
| ATG16L2 | NM_033388 | rs11235604 | Faux-sens | C | T |
| | | rs77419620 | Faux-sens | G | C |
| CALCOCO2 | NM_005831 | rs62621985 | Intron | G | A |
| | | rs2303015 | Faux-sens | A | G |
| | | rs550510 | Faux-sens | G | A |
| OPTN | NM_021980 | rs3829923 | 5' promoteur | C | T |
| CHMP2B | | rs63751126 | Intron | A | C |
| DRAM1 | NM_018370 | rs144227051 | | TC | Del |
| | | rs34935592 | eQTL ; 5' promoteur | A | C |
| | | rs55649039 | eQTL ; 5' promoteur | C | A |
| | | rs7960472 | eQTL ; 5' promoteur | C | G |
| | | rs112627014 | eQTL ; Intron | G | A |
| GABARAPL3 | NR_028287 | rs6496667 | eQTL ; 5' promoteur | C | A |
| IRGM | NM_001145805 | rs13361189 | eQTL | T | C |
| | | rs4958847 | eQTL | G | A |
| | | rs72553867 | Faux-sens | C | A |
| KIAA0226 | NM_001145642 | rs112632845 | Faux-sens | C | T |
| | | rs61742251 | Faux-sens | T | C |
| | | rs61746427 | Faux-sens | C | T |
| | | rs61746429 | Faux-sens | C | T |
| MAP1 LC3A | NM_181509 | rs72497006 | eQTL ; 5'promoteur | G | A |
| | | rs11167234 | eQTL ; 5' promoteur | T | A |
| | | rs6087598 | eQTL ; Intron | G | A |
| | | rs6088618 | eQTL ; 5' promoteur | G | A |
| MAP1LC3B2 | NM_001085481 | rs2089222 | eQTL intron | G | A |
| NBR1 | NM_005899 | rs142510229 | Faux-sens | A | G |
| | | rs34372250 | Faux-sens | A | G |
| OPTN | NM_021980 | rs11258194 | Faux-sens | T | A |
| | | rs2244380 | eQTL ; Intron | C/G | A |
| | | rs267606928 | Faux-sens,stop | C | T |
| | | rs267606929 | Faux-sens | A | G |
| | | rs28939688 | Faux-sens | G | A |
| | | rs72638765 | 3'UTR | A | G |
| | | rs75654767 | Faux-sens | G | A |
| | | rs765884 | eQTL ; Intron | T | C |
| PIK3C3 | NM_002647 | rs3813065 | eQTL ; 5' promoteur | C | T |
| | | rs4121817 | eQTL ; Intron | G | A |
| PIK3R4 | NM_014602 | rs111457529 | 3' | TAT | DEL |
| | | rs60201446 | eQTL ; Intron | T | C |
| RB1CC1 | NM_014781 | rs148814601 | Faux-sens | C | T |
| | | rs207469174 | Intron | G | T |
| | | rs77653001 | Faux-sens | C | T |
| SQSTM1 | NM_001142299 | rs104893941 | Faux-sens | C | T |
| | | rs10516140 | eQTL ; Intron | A | C |
| | | rs207466647 | Intron | A | G |
| ULK1 | NM_003565 | rs11546871 | Faux-sens | C | T |
| | | rs12303764 | Intron | T | G |
| ULK2 | NM_014683 | rs150122 | Faux-sens | G | A |
| | | rs157397 | Synonyme | T | C |
| | | rs281357 | eQTL ; Intron | T | C |
| | | rs34670978 | Faux-sens | G | A |
| UVRAG | NM_003369 | rs17134573 | eQTL ; Intron | G | A |
| | | rs192841804 | Faux-sens | A | T |
| | | rs594826 | Intron | C | T |
| | | rs7116263 | eQTL ; Intron | C | G |
| WIPI1 | NM_017983 | rs2909207 | eQTL ; Intron | T | C |
| | | rs883541 | Faux-sens | G | A |

| | | | | | |
|---|---|---|---|---|---|
| DEL : délétion | | | | | |

### Exemple 3 : Génotypage à haut débit par spectrométrie de masse Sequenom^{R}

### Matériels et Méthodes :

Pour chaque échantillon, un total de 50 ng d'ADN a été utilisé pour génotyper des multiplexes de 36 SNP selon la technique de génotypage MassARRAY (Sequenom^{R}). Brièvement, une région d'intérêt est amplifiée par PCR et une extension d'amorce d'une base est effectuée au niveau de la mutation. La spectrométrie de masse MALDI-TOF détecte la mutation par la masse de la base ajoutée de type sauvage ou mutée.

### Méthodes associées avec les analyses statistiques.

Le Logiciel GraphPad Prism7, (La Jolla California USA, www.graphpad.com) est utilisé pour l'analyse de données. Les fréquences alléliques et génotypiques de la cohorte de « *patients »* atteinte d'une myofasciite à macrophages persistante et de la cohorte « *sain* », ne présentant pas de signes cliniques habituellement associées à une myofasciite à macrophages persistante, ont été déterminées par comptage direct. Les fréquences observées ont été comparées avec les fréquences attendues et testées pour l'équilibre de Hardy-Weinberg. Les différences dans les fréquences alléliques et génotypiques entre les populations de patients et la population contrôle ont été mesurées en utilisant le test du Chi-carré et le test exact de Fischer. P <0,05 a été considéré comme statistiquement significatif.

### Résultats :

7 SNPs situés dans 6 gènes de l'autophagie ont été identifiés (Figure 1). Ils sont listés dans le Tableau 3 ci-dessous. Ces SNPs représentent soit des mutations ponctuelles (ATG2A, ATG10) soit des SNP synonymes eQTL rapportées dans les bases de données GTEX comme réduisant l'expression de l'allèle muté. Comme indiqué dans le Figure 2, le niveau d'expression de chaque gène associé à un SNP de type eQTL est significativement inhibé. Clairement, ces SNPs modulent l'expression des gènes associés malgré le fait qu'ils soient localisés dans les régions non codantes et n'induise donc pas de changement dans la séquence d'acide aminé de la protéine elle-même. Les 6 gènes identifiés ici, ayant 7 SNPs, sont plus particulièrement impliqués dans la formation de l'autophagosome, participant ainsi à la solubilisation intracellulaire des particules minérales (cf. Figure 3).

**Tableau 3 : 7 SNPs identifiés dans 6 gènes associés à une myofasciite à macrophages persistante**

| **Gène** | **SNP (rs)** | **Type de variante** | **Allèle(s)** | **p-val** |
|---|---|---|---|---|
| *ATG2A* | rs61746812 | Non-synonyme, V664M | T | < 0,0001 |
| *ATG9B* | rs2373927 | eQTL | G | 0,0391 |
| *ATG10* | rs3734114 | Non-synonyme, S62P | C | 0,0099 |
| *ATG10* | rs111249277 | eQTL/Intronique | AA* | 0,0139 |
| *ULK2* | rs157397 | Synonyme | T | 0,0005 |
| *IRGM* | rs4958847 | eQTL | A | 0,0135 |
| *MAP1LC3A* | rs6087598 | eQTL/Intronique | AA* | 0,0088 |

| | | | | |
|---|---|---|---|---|
| *le SNP est homozygote ; les deux loci doivent avoir l'allèle | | | | |

Ces SNPs sont associés à une myofasciite à macrophages persistante (Figure 4), elle-même due à une exposition aux adjuvants aluminiques. Plus particulièrement, le risque de développer une myofasciite à macrophages persistante augmente en fonction du nombre d'allèles présente chez un individu. Lorsqu'au moins deux des allèles (Tableau 3) sont présents parmi les 7 identifiés, le risque de développer une myofasciite à macrophages est fortement augmenté (Figure 4).

### REFERENCES

Agmon-Levin N et al. Immunol Res ; 60(2-3):376-83, 2014
Asiello and Baeumner, Lab Chip ; 11(8): 1420-1430, 2011
Authier et al., Arthritis Rheum ; 48(2): 569-70, 2003
Brest et al., Curr Mol Med. ; 10(5) : 486-502, 2010
Brest et al., Nat Genet. ; 43(3) :242-5, 2011
Brest et al., Autophagy ; 7(7) :786-7, 2011
Bentley et al., Nature ; 456: 53-59, 2008
Clarke et al., Nat Nanotechnol ; 4: 265-270, 2009
Couette et al., J Inorg Biochem ; 103(11): 1571-8, 2009
Crepeaux et al., Toxicology ; 28(375): 48-57, 2016
Garner et al., Genet Epidemiol. ; 31(4):288-95, 2007
Gherardi et al., Brain ; 124(9): 1821-31, 2001
Gherardi & Authier, Immunol Allergy Clin North Am. ; 23(4): 699-712, 2003
Eid et al., Science ; 323: 133-138, 2009
Harris et al., Science ; 320: 106-109, 2008
Henault et al., Immunity ; 14; 37(6): 986-997, 2012
Jakobsdottir et al., PLoS Genet ; 5(2):e1000337, 2009
Margulies et al., Nature ; 437: 376-380, 2005
Martinez et al., Proc Natl Acad Sci USA ; 18; 108(42):17396-401, 2011
McKernan et al., Genome Res ; 19: 1527-1541, 2009
Passeri et al., J Inorg Biochem ; 105(11): 1457-63, 2011
Romao et al., J Cell Biol ; 9; 203(5):757-66, 2013
Rothberg et al., Nature ; 475: 348-352, 2011
Sanjuan et al., Nature ; 20;450(7173):1253-7, 2007
Shibutani and Yoshimori, Cell Res ; 24(1):58-68, 2014
Van der Gucht et al., J Nucl Med ; 58(3): 492-498, 2017
Xie et al., Int J Cancer. ; 139(7): 1564-73, 2016
Zhang et al., J Immunol Res. ; 2015: 153132, 2015
WHO Vaccine Safety Advisory Committee. Wkly Epidemiol Rec ; 74: 338-40, 1999

## Revendications

1. Méthode permettant de prévoir le risque de développer une myofasciite à macrophages persistante, chez un sujet humain, comprenant :
a) la détermination de la présence ou l'absence du polymorphisme nucléotidique (SNP) rs61746812 du gène AGT2A dans un échantillon biologique; et
b) la détermination qu'il y a un risque lorsque ledit polymorphisme nucléotidique est détecté dans l'étape a).

2. Méthode de diagnostic chez un sujet humain atteint d'une myofasciite à macrophages persistante, comprenant :
a) la détermination de la présence ou l'absence du polymorphisme nucléotidique rs61746812 du gène AGT2A dans un échantillon biologique ; et
b) la détermination, lorsque ledit polymorphisme nucléotidique est détecté, que ledit sujet est atteint d'une myofasciite à macrophages.

3. Méthode selon l'une des revendications 1 ou 2 **caractérisée** en ce la présence ou l'absence d'au moins un polymorphisme nucléotidique supplémentaire choisi parmi rs2373927 du gène ATG9B, rs3734114 ou rs111249277 du gène ATG10, rs157397 du gène ULK2, rs4958847 du gène IGRM, ou rs6087598 du gène MAP1LC3A est détecté à l'étape a).

4. Méthode selon la revendication 3 **caractérisée en ce que** au moins trois polymorphismes nucléotidiques sont détectés à l'étape a).

5. Méthode selon l'une des revendications 3 ou 4 comprenant la détermination du risque à l'étape b) selon le nombre de polymorphismes identifiés à l'étape a).

6. Méthode selon l'une des revendications 1 à 5, **caractérisée en ce que** l'échantillon biologique est un fluide biologique, de préférence du sang, du plasma, de la lymphe ou de la salive.

7. Méthode selon l'une des revendications 1 à 6, dans laquelle la myofasciite à macrophages biopersistante est associée à une exposition à un composé à effet adjuvant, de façon préférentielle un sel d'aluminium, de façon encore plus préférentielle l'oxy-hydroxyde d'aluminium ou le hydroxy-phosphate d'aluminium.

8. Méthode permettant de prévoir le risque ou de diagnostiquer une myofasciite à macrophages persistante chez un sujet selon l'une des revendications 1 à 6 **caractérisée en ce que** l'étape a) comprend :
a) l'isolement d'un acide nucléique dudit sujet ;
b) optionnellement, l'amplification dudit l'acide nucléique ;
c) la discrimination allélique ; et
d) la détermination de la présence ou de l'absence d'au moins un polymorphisme nucléotidique selon l'étape c).

9. Méthode selon la revendication 8 dans laquelle l'amplification à l'étape b) est faite par PCR avec des amorces de 12 à 30 nucléotides contigus permettant l'amplification d'un SNP parmi rs61746812, rs2373927, rs3734114, rs111249277, rs157397, rs4958847, ou rs6087598, ou par amplification isotherme dudit SNP et/ou l'étape c) est faite par hybridation d'une sonde, par amplification, par séquençage, par spectrométrie de masse, par transfert sur membrane par la technique de Southern, ou par une combinaison de ces techniques.

10. Kit pour la détection de la présence ou l'absence du polymorphisme nucléotidique (SNP) rs61746812 du gène AGT2A comprenant au moins un réactif reconnaissant la séquence allélique spécifique choisi parmi un couple d'amorces et une sonde reconnaissant la séquence allélique spécifique.


SEQUENCE LISTING

<110>  ASSISTANCE PUBLIQUE - HOPITAUX DE PARIS
       ASSOCIATION FRANCAISE CONTRE LES MYOPATHIES
       UNIVERSITE DE NICE SOPHIA ANTIPOLIS
       CENTRE NATIONAL DE LA RECHERCHE SCIENTIFIQUE (CNRS)
       UNIVERSITE PARIS EST CRETEIL VAL DE MARNE
       INSTITUT NATIONAL DE LA SANTE ET DE LA RECHERCHE MEDICALE
       (INSERM)

<120>  Méthode de détermination de la susceptibilité individuelle

<130>  B249723D33654

<160>  14

<170>  PatentIn version 3.5

<210>  1
<211>  51
<212>  DNA
<213>  Homo sapiens

<400>  1
tccagccgaa gctgctcagc ccgcacggcc tggcccgccc aggggtccgg c                51


<210>  2
<211>  51
<212>  DNA
<213>  Homo sapiens

<400>  2
tccagccgaa gctgctcagc ccgcatggcc tggcccgccc aggggtccgg c                51


<210>  3
<211>  51
<212>  DNA
<213>  Homo sapiens

<400>  3
taggcagagg tccctgcagc cttcctcagt gttttgtgtc tgggtacttg a                51


<210>  4
<211>  51
<212>  DNA
<213>  Homo sapiens

<400>  4
taggcagagg tccctgcagc cttccgcagt gttttgtgtc tgggtacttg a                51


<210>  5
<211>  51
<212>  DNA
<213>  Homo sapiens

<400>  5
gtctgtgatg tcacatctag gagcacctac ccatggacag acatgtcttc c                51

<210>  6
<211>  51
<212>  DNA
<213>  Homo sapiens

<400>  6
gtctgtgatg tcacatctag gagcatctac ccatggacag acatgtcttc c                51


<210>  7
<211>  51
<212>  DNA
<213>  Homo sapiens

<400>  7
cgggtccccg ccggtaactg caggcctgtg ctggccgccg cgagcgggga a                51


<210>  8
<211>  51
<212>  DNA
<213>  Homo sapiens

<400>  8
cgggtccccg ccggtaactg caggcatgtg ctggccgccg cgagcgggga a                51


<210>  9
<211>  51
<212>  DNA
<213>  Homo sapiens

<400>  9
gctcaagaaa aggatggcta aaaaacgctt ctgtaacaag aaatgagaat g                51


<210>  10
<211>  51
<212>  DNA
<213>  Homo sapiens

<400>  10
gctcaagaaa aggatggcta aaaaatgctt ctgtaacaag aaatgagaat g                51


<210>  11
<211>  51
<212>  DNA
<213>  Homo sapiens

<400>  11
ctttatagat ttcattgccc aatatggcta aataatgcac tactccctaa a                51


<210>  12
<211>  51
<212>  DNA
<213>  Homo sapiens

<400>  12
ctttatagat ttcattgccc aatatagcta aataatgcac tactccctaa a                51

<210>  13
<211>  51
<212>  DNA
<213>  Homo sapiens

<400>  13
cttgaaggtg ggtggcggag gttgcggtac gccaatattg tgccgttgca c                51


<210>  14
<211>  51
<212>  DNA
<213>  Homo sapiens

<400>  14
cttgaaggtg ggtggcggag gttgcagtac gccaatattg tgccgttgca c                51